# FASCICULE DE BREVET EUROPEEN

(11) **EP 1 488 715 B1**
(45) Date de publication et mention de la délivrance du brevet: **09.08.2006**
(21) Numéro de dépôt: 04356099.4
(22) Date de dépôt: 16.06.2004
(51) Int. Cl.: A43B 13/14, A43B 7/14, A43B 13/12, A43B 13/36, A43B 3/24, A61F 5/01

(54) **Chaussure post-opératoire évolutive**
Anpassbares postoperatives Schuhwerk
Upgradable post-operative footwear

(30) Priorité: 20.06.2003 FR 0307502
(43) Date de publication de la demande: 22.12.2004
(73) Titulaire: Ormihl-Danet, 69100 Villeurbanne (FR); Francon, Henri, 69380 Dommartin (FR); Peyrot, Jacques, 69160 Tassin la Demi Lune (FR); Augoyard, Marc, 69160 Tassin la Demi Lune (FR); Pfaiffer, Eric, 69370 Saint Didier au Mont D'Or (FR)
(72) Inventeur: Pfaiffer, Eric, 69370 Saint Didier au Mont d'Or (FR); Didelot, Raphael, 69360 Serezin du Rhone (FR); Peyrot, Jacques, 69160 Tassin la Demi Lune (FR); Francon, Henri, 69380 Dommartin (FR); Augoyard, Marc, 69160 Tassin la Demi Lune (FR)
(74) Mandataire: Bratel, Gérard

(56) Documents cités:
- DE-A- 19 930 233
- FR-A- 1 288 897
- US-A- 4 726 127
- US-A- 4 821 432
- US-B1- 6 277 087

## Description

La présente invention concerne une chaussure évolutive à visée médico-chirurgicale. La chaussure permet en particulier d'appareiller des patients venant de subir une intervention chirurgicale concernant l'avant-pied ou l'arrière-pied, et notamment l'opération courante dite de l'hallus valgus (déformation du gros orteil). Cette chaussure permet, grâce à ses possibilités de transformation aisée et adaptée, d'appareiller le patient pratiquement dès sa sortie du bloc opératoire, et ce jusqu'à la fin de sa rééducation.

En l'état actuel de la technique, il n'existe aucune chaussure post-opératoire utilisable depuis l'opération jusqu'à la fin de la convalescence, ce qui oblige le patient à acquérir et utiliser, successivement, deux ou plusieurs chaussures distinctes, adaptées chacune à une phase particulière de la période post-opératoire.

La plupart des chaussures post-opératoires, actuellement connues, possèdent en effet une structure figée, ou seulement adaptable dans une mesure assez limitée.

En particulier, on connaît les chaussures qui possèdent, sous leur semelle, une cale de forme plate pour la décharge de l'avant-pied, ou un tampon de forme arrondie pour faciliter le "déroulement" du pied lors de la marche. On peut ici se référer aux documents suivants :
- pour les cales : brevets EP 0248964 (ou son équivalent US 4726127), EP 0557409, US 3198192, US 6282818 et DE 19930233, ainsi que le modèle d'utilité allemand DE 29905144 U ;
- pour les tampons arrondis : brevet US 4821432, et aussi le brevet allemand précité DE 19930233.

Dans la plupart des réalisations selon ces documents, la cale ou le tampon est fixe, du fait de sa réalisation d'une seule pièce avec la semelle, ou d'une fixation définitive par collage sous la semelle. Un montage amovible et/ou réglable en position longitudinale, pour une cale ou un tampon, est cependant enseigné par le brevet US 4821432 et par le brevet allemand DE 19930233 précédemment mentionnés.

Par ailleurs, il existe des chaussures post-opératoires dont la semelle peut être rigidifiée par une plaque inférieure ou intermédiaire, éventuellement amovible - voir les brevets US 5569173, US 5569171, US 6 277087, qui décrit le préambule de la revendication 1, et US 6282818.

Malgré les nombreuses propositions antérieures existantes, telles que celles indiquées ci-dessus, on ne connaît pas de chaussures post-opératoires conçues de façon véritablement modulaire, en vue d'une adaptation à toutes les phases post-opératoires, autrement dit pendant toute la période de rééducation, tout en offrant des possibilités de réglage étendues pour une adaptation à tous les volumes de pied et à la plupart des cas pathologiques rencontrés.

La présente invention vise à remédier à ces inconvénients, en fournissant une chaussure post-opératoire polyvalente, pouvant être utilisée dans la phase médicale comme dans la phase de réadaptation du patient, donc depuis l'intervention chirurgicale jusqu'à la fin de la convalescence.

A cet effet, l'invention a pour objet une chaussure post-opératoire évolutive, qui comprend essentiellement, d'une part, un corps de base, avec une semelle semi-rigide et une tige de chaussure munie de moyens de fermeture, et d'autre part, un ensemble d'accessoires adaptables de façon démontable et interchangeable sous la semelle du corps de base, lesdits accessoires comprenant au moins une plaque métallique de rigidification, une cale de décharge de l'avant-pied, un tampon de forme arrondie et un talon, utilisables séparément ou en combinaison, des moyens étant prévus pour la fixation amovible de ces accessoires sous la semelle de base et/ou entre eux la plaque métallique de rigidification possédant au moins une glissière longitudinale, prévue pour la fixation amovible et réglable en position longitudinale, sous cette plaque métallique, de la cale de décharge de l'avant-pied ou du tampon de forme arrondie, au moyen d'autres vis.

Ainsi, l'invention propose une chaussure post-opératoire de structure modulaire, qui la rend réellement "évolutive", au sens d'une possibilité de transformation importante et adaptée de cette chaussure, lui permettant de répondre à tous les cas d'utilisation liés à l'avant-pied ou à l'arrière-pied, et de s'adapter, tant par sa configuration variable que par une rigidité ajustable, aux différentes phases consécutives à l'opération, et ceci jusqu'à ce que le patient puisse reprendre un chaussage classique.

En particulier, la plaque métallique de rigidification est adaptée sous la semelle du corps de base de cette chaussure post-opératoire, dans la première phase de rééducation, cette plaque étant utilisable seule ou en combinaison avec la cale de décharge de l'avant-pied ou avec le tampon de forme arrondie.

On comprend que ces dispositions permettent, simultanément, la fixation au choix et amovible de la cale de décharge de l'avant-pied ou du tampon permettant un meilleur "déroulement" du pied lors de la marche, et le réglage de la position longitudinale de l'un ou l'autre de ces accessoires.

Pour l'adaptation notamment de ladite plaque métallique, la semelle semi-rigide du corps de base de la chaussure post-opératoire comporte avantageusement des inserts métalliques taraudés, en correspondance avec des trous de la plaque métallique, des vis de fixation de cette plaque traversant les trous de celle-ci et étant serrées dans les inserts taraudés de la semelle du corps de base.

La plaque métallique de rigidification est aussi utilisable seule, c'est-à-dire sans autre accessoire fixé sous elle ; à cet effet, ladite plaque métallique porte avantageusement, sur sa face inférieure, un revêtement en une matière du genre caoutchouc, pouvant être du caoutchouc, de la gomme naturelle, une matière élastomérique.

Par contre, durant la deuxième phase de rééducation, la plaque métallique de rigidification est démontée, afin de rendre sa souplesse initiale à la semelle semi-rigide du corps de base ; la cale ou le tampon est enlevé en même temps, s'il en a été fait usage. Il devient alors possible de positionner, sous la semelle du corps de base et plus particulièrement à l'arrière de cette semelle, le talon qui permet la marche dans des conditions pratiquement normales. La fixation du talon est réalisable au moyen de vis serrées dans les inserts taraudés déjà prévus dans la partie arrière de la semelle du corps de base, pour la fixation de la semelle métallique comme indiqué ci-dessus.

A titre facultatif et complémentaire, la chaussure post-opératoire évolutive, objet de la présente invention, peut encore comporter d'autres accessoires amovibles prenant place au-dessus de la semelle du corps de base, ou en avant de celle-ci, en particulier :
- une semelle interne élargie, pour le confort du patient et l'adaptation de la chaussure post-opératoire à un pied enflé ou à des pansements plus ou moins volumineux ;
- un butoir se fixant à l'avant du corps de base, pour protéger l'avant-pied de tous les chocs.

L'adaptation de ce butoir amovible est réalisable au moyen de vis, en mettant à profit les inserts taraudés déjà prévus dans la partie avant de la semelle du corps de base, pour la fixation de la semelle métallique comme exposé ci-dessus.

On notera que la corps de base de la chaussure post-opératoire, ses divers accessoires amovibles et leurs moyens de fixation (vis) peuvent être livrés de façon groupée, comme un produit unique répondant à tous les besoins, ce qui rend la solution objet de l'invention particulièrement simple et économique, tant pour le patient que pour les organismes (Sécurité Sociale, mutuelles) appelés à prendre en charge les frais de convalescence et de rééducation.

L'invention sera mieux comprise à l'aide de la description qui suit, en référence au dessin schématique annexé représentant, à titre d'exemple, une forme d'exécution de cette chaussure post-opératoire évolutive :
Figure 1 représente, en perspective éclatée, le corps de base et divers accessoires de la chaussure post-opératoire : semelle élargie, plaque métallique, cale, butoir ;
Figure 2 représente, en perspective, le tampon en tant qu'autre accessoire ;
Figure 3 représente, en perspective, le talon en tant que dernier accessoire ;
Figure 4 est une vue de côté avec coupe partielle illustrant une utilisation de cette chaussure post-opératoire ;
Figure 5 est une vue de côté avec coupe partielle illustrant une autre utilisation de la même chaussure ;
Figure 6 est une vue de côté avec coupe partielle illustrant une dernière utilisation de ladite chaussure.

La chaussure post-opératoire évolutive, représentée au dessin, comprend un corps de base 2, avec une semelle 3 et une tige de chaussure 4. La semelle 3 est réalisée en une matière semi-rigide, telle que gomme naturelle, et elle présente une face inférieure 5 plane. La tige de chaussure 4 est réalisée par exemple en une matière textile, telle que du coton tissé, et elle est avantageusement rembourrée intérieurement par de la mousse, par exemple de la mousse de polyester. Cette tige 4 est du genre "sandale" laissant en particulier découverte la partie avant, et aussi éventuellement la partie arrière du pied du patient ; elle comporte des moyens de fermeture, ici réalisés comme des sangles 6 auto-aggripantes, qui permettent une fixation rapide sur le pied et un réglage aisé en fonction de la grosseur du pied, la marge de réglage étant importante pour tenir compte de l'éventualité d'oedèmes ou de pansements volumineux. La tige de chaussure 4 peut comporter aussi des renforts ou un contrefort.

Dans la semelle 3 du corps de base 2 sont noyés des inserts taraudés 7, métalliques, au nombre total de quatre, avec deux premiers inserts 7 situés dans la partie arrière de la semelle 3, et deux autres inserts 7 situés dans la partie avant de cette semelle 3. Chaque insert 7 est accessible depuis la face inférieure 5 de la semelle 3, et il peut ainsi recevoir une vis de fixation, comme détaillé ci-après.

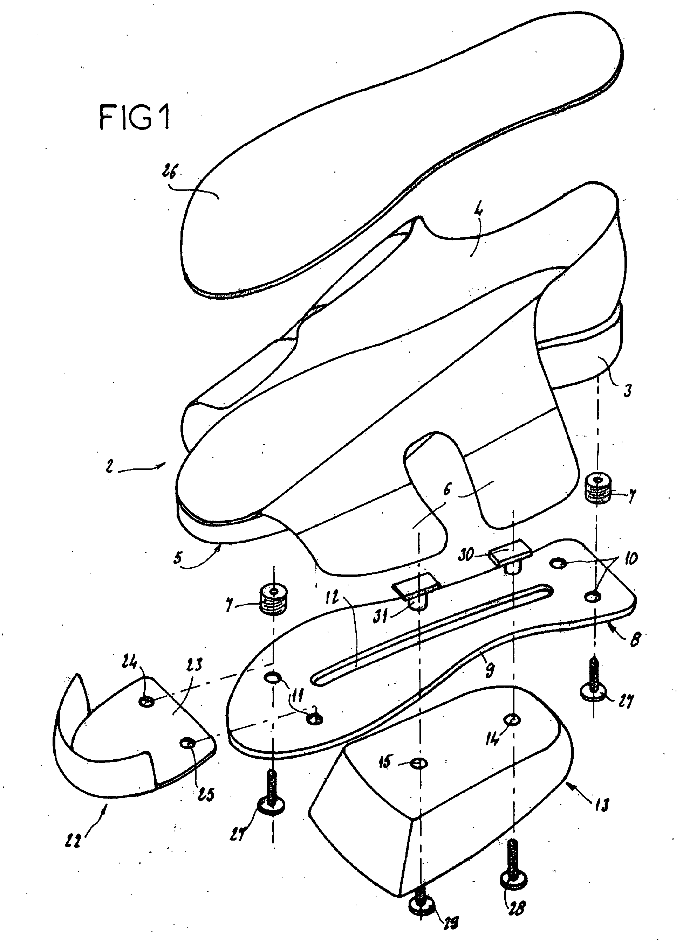

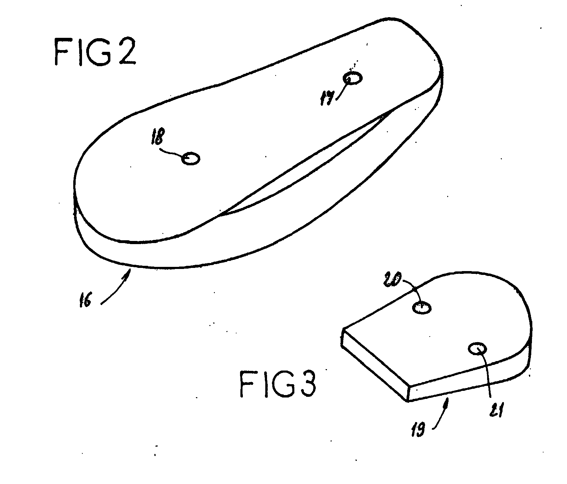

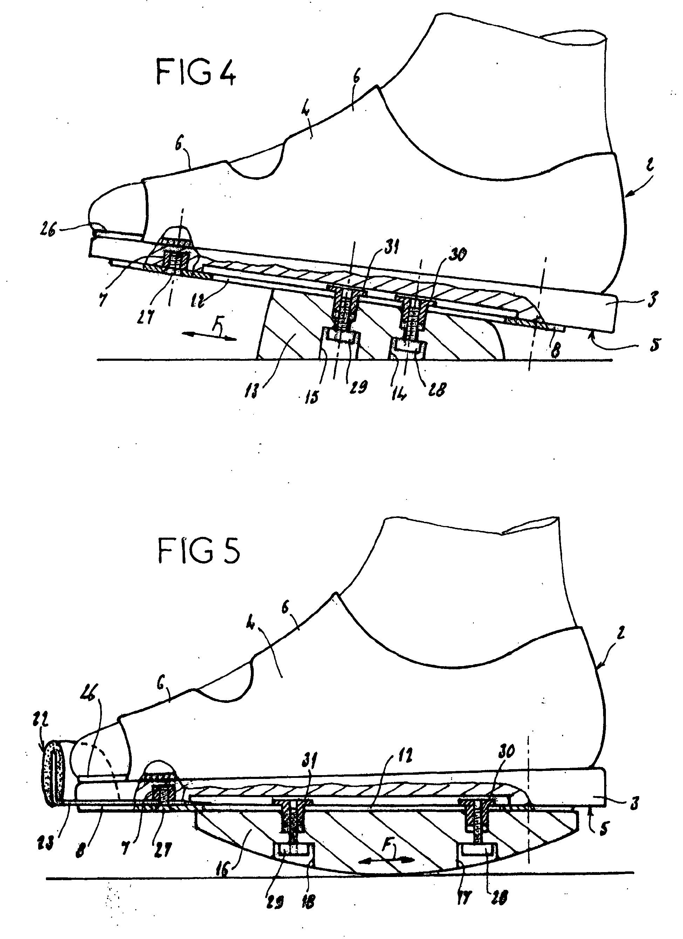

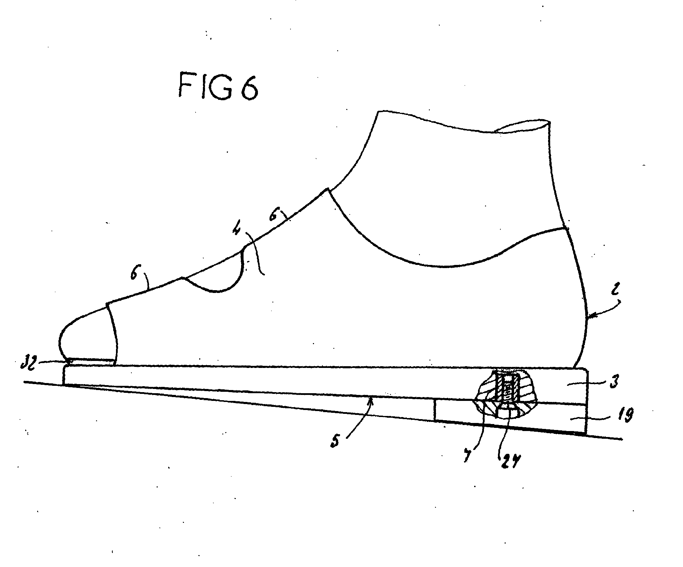

Le corps de base 2, précédemment décrit, est prévu pour recevoir divers accessoires amovibles et interchangeables, dont certains sont montrés sur la figure 1, et d'autres sont représentés séparément sur les figures 2 et 3. Ces accessoires comprennent :
a) Une plaque métallique 8 de rigidification, notamment une plaque en aluminium, dont le contour 9 s'inscrit dans celui de la semelle 3 du corps de base 2. La plaque métallique 8 comporte deux trous 10 dans sa partie arrière, et deux autres trous 11 dans sa partie avant, les deux trous 10 ou 11 de chaque paire étant symétriques par rapport à l'axe médian longitudinal de cette plaque métallique 8. La plaque métallique 8 possède aussi une glissière longitudinale unique, réalisée sous la forme d'une fente longitudinale 12 qui s'étend sensiblement suivant l'axe médian de ladite plaque métallique 8.
b) Une cale 13 de décharge de l'avant-pied, de profil sensiblement quadrangulaire (voir aussi la figure 4), qui est pourvue de deux trous 14 et 15 percés l'un dans la partie arrière, et l'autre dans la partie avant de cette cale 13, dans son plan médian longitudinal.
c) Un tampon 16 de profil arrondi (voir aussi la figure 5), qui est lui aussi pourvu de deux trous 17 et 18 percés l'un dans la partie arrière, et l'autre dans la partie avant de ce tampon 16, dans son plan médian longitudinal.
d) Un talon 19 de petite dimension (voir aussi la figure 6), qui est pourvu de deux trous 20 et 21 disposés symétriquement par rapport à son plan médian longitudinal, à un écartement égal à celui des premiers inserts 7 situés dans la partie arrière de la semelle 3 du corps de base 2.
e) Un butoir 22 avec une embase de fixation 23 pourvue de deux trous 24 et 25, disposés symétriquement par rapport à son plan médian longitudinal, à un écartement égal à celui des inserts 7 situés dans la partie avant de la semelle 3 du corps de base 2.
f) Une semelle interne élargie 26, c'est-à-dire de largeur supérieure à celle de la semelle 3 du corps de base 2. La cale 13, le tampon 16, le talon 19 et le butoir 22 sont réalisés en une matière synthétique relativement rigide, notamment en polypropylène. Le butoir 22 comporte avantageusement un capitonnage intérieur en mousse amovible. La semelle interne élargie 26, est réalisable en ABS, et elle est avantageusement pourvue, sur sa face inférieure, d'un revêtement auto-aggripant.

L'utilisation de la chaussure post-opératoire, précédemment décrite, est la suivante :
Durant une première phase de rééducation qui suit immédiatement l'intervention chirurgicale du pied, la rigidité de la chaussure est assurée au moyen de la plaque métallique 8, appliquée contre la face inférieure 5 de la semelle 3 du corps de base 2, et fixée contre cette face inférieure 5 au moyen de quatre vis 27 - voir les figures 4 et 5. Plus particulièrement, deux premières vis de fixation 27 sont engagées au travers des deux trous 10 de la partie arrière de la plaque métallique 8, et sont serrées dans les taraudages des deux inserts 7 de la partie arrière de la semelle 3 ; deux autres vis de fixation 27 sont engagées au travers des deux trous 11 de la partie avant de la plaque métallique 8, et sont serrées dans les taraudages des deux inserts 7 de la partie avant de la semelle 3.

La chaussure post-opératoire, ainsi rigidifiée par la plaque métallique 8, est utilisable dans cet état, ou équipée en outre de la cale 13 ou du tampon 16, fixé sous ladite plaque métallique 8.

La figure 4 illustre l'utilisation de la chaussure avec la cale 13, destinée à la décharge de l'avant-pied. Cette cale est montée sous la partie médiane de la plaque métallique 8, et elle est fixée au moyen de deux vis 28 et 29, engagées respectivement au travers de ses deux trous 14 et 15, et vissées dans des coulisseaux taraudés respectifs 30 et 31 placés dans la fente longitudinale 12 de la plaque métallique 8, et déplaçables le long de cette fente 12. La cale 13 peut ainsi être fixée, sous la chaussure, dans une position réglable longitudinalement, comme suggéré par une double flèche F.

La figure 5 illustre l'utilisation de la chaussure avec le tampon 16, dont le profil arrondi permet un meilleur "déroulement" du pied lors de la marche. Comme la cale 13, le tampon 16 est fixé sous la plaque métallique 8 au moyen de deux vis 28 et 29, qui traversent ici les trous 17 et 18 du tampon 16 et coopèrent avec les coulisseaux 30 et 31, la position du tampon 16 étant réglable longitudinalement (double flèche F).

Dans la première phase d'utilisation, la chaussure peut aussi être équipée du butoir 22, comme l'illustre la figure 5. L'embase 23 du butoir 22 est engagée entre la semelle 3 du corps de base 2 et la plaque métallique 8, à l'avant de la chaussure; les trous 24 et 25 de l'embase 23 sont traversés par les vis 27 serrées dans les deux inserts taraudés 7 prévus dans la partie avant de la semelle 3. La mise en place du butoir 22 garantit l'avant-pied de tous les chocs.

La semelle élargie 26 peut aussi être mise en place, dans cette première phase d'utilisation, en étant posée sur la semelle 3 du corps de base 2, à l'intérieur de la chaussure, le revêtement inférieur auto-aggripant de ladite semelle élargie 26 assurant son maintien en position. La semelle 3 du corps de base 2 se trouve ainsi élargie, pour recevoir un pied enflé ou recouvert de pansements. La souplesse de la tige de chaussure 4, et la longueur des sangles 6, sont adaptées pour envelopper le volume important résultant de la semelle élargie 26 et/ou d'un pied enflé ou recouvert de pansements, en permettant toujours la fermeture de la chaussure.

Durant une deuxième phase de rééducation, la plaque métallique 8 de rigidification est démontée, par desserrage des vis 27, afin de rendre sa souplesse initiale à la semelle 3 du corps de base 2. La cale 13 ou le tampon 16, et le cas échéant le butoir 22, sont enlevés en même temps. Le talon 19 sera alors positionné à l'arrière de la chaussure, sous la semelle 3 du corps de base 2, en utilisant les deux inserts taraudés 7 de la partie arrière de cette semelle 3, pour y serrer deux vis 27 engagées aussi au travers des deux trous 20 et 21 du talon 19 - voir figure 6.

Dans cette deuxième phase d'utilisation, la semelle élargie 26 aura elle aussi été retirée, pour redonner un volume normal à la chaussure, l'oedème ayant très fortement diminué et les pansements étant devenus très peu encombrants. Une nouvelle semelle interne 32, de contour coïncidant avec celui de la semelle 3 du corps de base 2, peut alors prendre la place de la semelle élargie.

En cas de déséquilibre de la statique, de petits éléments pronateurs ou supinateurs (non représentés) pourront être positionnés sous le talon 19.

La mise en place et le retrait des divers accessoires, et le réglage de position éventuel de ceux-ci, sont aisément réalisables au moyen d'un outil adapté, tel que clé à alène, permettant de manoeuvrer les vis 27, 28 et 29, dans le sens du serrage ou de desserrage.

Bien entendu, cette chaussure post-opératoire et ses divers accessoires seront disponibles pour un pied droit et un pied gauche et en toutes pointures.

L'on ne s'éloignerait pas du cadre de l'invention, telle que définie dans les revendications annexées, quels que soient :
- les matériaux constitutifs des diverses parties ou accessoires de la chaussure, tels que la plaque de rigidification ;
- le détail des moyens de fermeture de la tige de chaussure (sangles, boucles, lacets...) ;
- les éventuels accessoires complémentaires adaptables sur la chaussure ;
- les utilisations de cette chaussure, tant comme chaussure post-opératoire, que comme chaussure adaptée à la pathologie globale du pied.

## Revendications

1. Chaussure post-opératoire évolutive, en particulier pour l'appareillage de patients ayant subi une intervention chirurgicale concernant l'avant-pied ou l'arrière-pied, ladite chaussure comprenant, d'une part, un corps de base (2), avec une semelle semi-rigide (3) et une tige de chaussure (4) munie de moyens de fermeture (6), et d'autre part, un ensemble d'accessoires adaptables de façon démontable et interchangeable sous la semelle (3) du corps de base (2), lesdits accessoires comprenant au moins une plaque métallique (8) de rigidification, une cale (13) de décharge de l'avant-pied, un tampon (16) de forme arrondie et un talon (19), utilisables séparément ou en combinaison, des moyens (7, 27 à 31) étant prévus pour la fixation amovible de ces accessoires (8, 13, 16, 19) sous la semelle (3) du corps de base (2) et/ou entre eux, **caractérisée en ce que** la plaque métallique (8) de rigidification possède au moins une glissière longitudinale (12), prévue pour la fixation amovible et réglable en position longitudinale, sous cette plaque métallique (8), de la cale (13) de décharge de l'avant-pied ou du tampon (16) de forme arrondie, au moyen de vis (28,29).

2. Chaussure post-opératoire selon la revendication 1, **caractérisée en ce que** la plaque métallique (8) de rigidification est une plaque en aluminium.

3. Chaussure post-opératoire selon la revendication 1 ou 2, **caractérisée en ce que**, pour l'adaptation notamment de la plaque métallique (8) de rigidification, la semelle semi-rigide (3) du corps de base (2) comporte des inserts métalliques taraudés (7), en correspondance avec des trous (10,11) de la plaque métallique (8), des vis de fixation (27) de cette plaque (8) traversant les trous (10,11) de celle-ci et étant serrées dans les inserts taraudés (7) de la semelle (3) du corps de base (2).

4. Chaussure post-opératoire selon la revendication 3, **caractérisée en ce que** sont prévus quatre inserts taraudés (7), avec deux inserts (7) situés dans la partie arrière de la semelle (3) du corps de base (2), et deux autres inserts (7) situés dans la partie avant de cette semelle (3), ces inserts (7) recevant respectivement quatre vis de fixation (27) de la plaque métallique (8) de rigidification.

5. Chaussure post-opératoire selon l'une quelconque des revendications 1 à 4, **caractérisée en ce que** la plaque métallique (8) de rigidification possède une glissière unique, sous la forme d'une fente longitudinale (12) s'étendant sensiblement suivant l'axe médian de ladite plaque métallique (8), la cale (13) de décharge de l'avant-pied et le tampon (16) de profil arrondi étant fixés l'une et l'autre au moyen de deux vis (28, 29) engagées dans cette fente longitudinale (12).

6. Chaussure post-opératoire selon l'une quelconque des revendications 1 à 5, **caractérisée en ce que** la plaque métallique (8) de rigidification porte, sur sa face inférieure, un revêtement en une matière du genre caoutchouc.

7. Chaussure post-opératoire selon la revendication 4, **caractérisée en ce que** la fixation du talon (19) est réalisée au moyen de vis (27) serrées dans les inserts taraudés (7) prévus dans la partie arrière de la semelle (3) du corps de base (2).

8. Chaussure post-opératoire selon l'une quelconque des revendications 1 à 7, **caractérisée en ce que** les accessoires que sont la cale (13) de décharge de l'avant-pied, le tampon (16) de forme arrondie, et le talon (19), sont réalisés en une matière synthétique relativement rigide, notamment en polypropylène.

9. Chaussure post-opératoire selon l'une quelconque des revendications 1 à 8, **caractérisée en ce qu'**elle comprend, comme accessoire amovible supplémentaire, un butoir (22) se fixant à l'avant du corps de base (2), pour protéger l'avant-pied des chocs.

10. Chaussure post-opératoire selon l'ensemble des revendications 4 et 9, **caractérisée en ce que** l'adaptation du butoir amovible (22) est réalisée au moyen de vis (27), serrées dans les inserts taraudés (7) prévus dans la partie avant de la semelle (3) du corps de base (2).

11. Chaussure post-opératoire selon l'une quelconque des revendications 1 à 10, **caractérisée en ce qu'**elle comprend encore une semelle interne élargie (26), amovible, prenant place au-dessus de la semelle (3) du corps de base (2).

## Claims

1. Adaptable post-operative shoe, in particular for equipping patients who have undergone a surgical intervention concerning the front or rear part of the foot, the said shoe comprising, firstly, a base body (2), with a semi-rigid sole (3) and shoe upper (4) provided with closure means (6), and secondly a set of accessories adaptable in demountable and interchangeable fashion under the sole (3) of the base body (2), the said accessories comprising at least one metal stiffening plate (8), a wedge (13) for relieving the front of the foot, a pad (16) of rounded shape and a heel (19), able to be used separately or in combination, means (7, 27 to 31) being provided for the removable fixing of these accessories (8, 13, 16, 19) under the sole (3) of the base body (2) and/or between them, **characterised in that** the metal stiffening plate (8) possesses at least one longitudinal runner (12), provided for the removable fixing, adjustable for longitudinal position, under this metal plate (8), of the wedge (13) for relieving the front part of the foot or of the pad (16) of rounded shape, by means of screws (28, 29).

2. Post-operative shoe according to claim 1, **characterised in that** the metal stiffening plate (8) is an aluminium plate.

3. Post-operative shoe according to claim 1 or 2, **characterised in that**, for the adaptation in particular of the metal stiffening plate (8), the semi-rigid sole (3) of the base body (2) comprises threaded metal inserts (7), matching the holes (10, 11) in the metal plate (8), fixing screws (27) for this plate (8) passing through the holes (10, 11) therein and being tightened in the threaded inserts (7) of the sole (3) of the raised body (2).

4. Post-operative shoe according to claim 3, **characterised in that** four threaded inserts (7) are provided, with two inserts (7) situated in the rear part of the sole (3) of the base body (2) and two other inserts (7) situated in the front part of this sole (3), these inserts (7) receiving respectively four fixing screws (27) for the metal stiffening plate (8).

5. Post-operative shoe according to any one of claims 1 to 4, **characterised in that** the metal stiffening plate (8) has a single runner, in the form of a longitudinal slot (12) extending substantially along the median axis of the said metal plate (8), the wedge (13) for relieving the front of the foot and the pad (16) with rounded profile being fixed to each other by means of two screws (28, 29) engaged in this longitudinal slot (12).

6. Post-operative shoe according to any one of claims 1 to 5, **characterised in that** the metal stiffening plate (8) carries, on its bottom face, a covering made from a material of the rubber type.

7. Post-operative shoe according to claim 4, **characterised in that** the heel (19) is fixed by means of screws (27) tightened in the threaded inserts (7) provided in the rear part of the sole (3) of the base body (2).

8. Post-operative shoe according to any one of claims 1 to 7, **characterised in that** the accessories consisting of the wedge (13) relieving the front of the foot, the pad (16) of rounded shape and the heel (19) are produced from a relatively rigid synthetic material, in particular polypropylene.

9. Post-operative shoe according to any one of claims 1 to 8, **characterised in that** it comprises, as a supplementary removable accessory, a buffer (22) fixed to the front of the base body (2) for protecting the front part of the foot from impacts.

10. Post-operative shoe according to claims 4 and 9 together, **characterised in that** the removable buffer (22) is fitted by means of screws (27) tightened in the threaded inserts (7) provided in the front part of the sole (3) of the base body (2).

11. Post-operative shoe according to any one of claims 1 to 10, **characterised in that** it also comprises a removable broadened internal sole (26), fitting above the sole (3) of the base body (2).

## Patentansprüche

1. Anpassbares postoperatives Schuhwerk, insbesondere für die Ausstattung von Patienten, bei denen ein chirurgischer Eingriff am vorderen Teil oder am hinteren Teil des Fußes ausgeführt wurde, wobei dieses Schuhwerk einerseits ein Basisteil (2) mit einer halbsteifen Sohle (3) und einen mit Verschlußvorrichtungen (6) versehenen Schuhschaft (4) umfasst und andererseits ein Ensemble von anpassbaren Zubehörteilen, die unter der Sohle (3) des Basisteils herausgenommen und ausgetauscht werden können, wobei diese Zubehörteile mindestens eine Metallplatte (8) zur Versteifung, einen Keil (13) zur Entlastung des Vorderfußes, ein abgerundetes Pufferteil (16) und einen Absatz (19) umfassen, die getrennt oder miteinander kombiniert eingesetzt werden können, wobei Teile (7, 27 bis 31) vorgesehen sind zur lösbaren Befestigung dieser Zubehörteile (8, 13, 16, 19) unter der Sohle (3) des Basisteils (2) und/oder untereinander, **dadurch gekennzeichnet, dass** die Metallplatte (8) zur Versteifung mindestens eine in Längsrichtung laufende Führungsbahn (12) aufweist, die für die mittels Schrauben (28, 29) lösbare und in Längsrichtung unter dieser Metallplatte (8) verstellbare Befestigung des Keils (13) zur Entlastung des Vorderfußes oder des abgerundeten Pufferteils (16) vorgesehen ist.

2. Postoperatives Schuhwerk nach Anspruch 1, **dadurch gekennzeichnet, dass** die Metallplatte (8) zur Versteifung eine Platte aus Aluminium ist.

3. Postoperatives Schuhwerk nach Anspruch 1 oder 2, **dadurch gekennzeichnet, dass** für die Anpassung insbesondere der Metallplatte (8) zur Versteifung die halbsteife Sohle (3) des Basisteils (2) metallische Einsätze (7) mit Innengewinde hat, die mit Löchern (10, 11) der Metallplatte (8) übereinstimmen, wobei Befestigungsschrauben (27) der Metallplatte (8) durch die Löcher (10, 11) dieser Platte gesteckt und mit den mit Innengewinden versehenen Einsätzen (7) der Sohle (3) des Basisteils (2) verschraubt werden.

4. Postoperatives Schuhwerk nach Anspruch 3, **dadurch gekennzeichnet, dass** vier Einsätze (7) mit Innengewinde vorgesehen sind, von denen zwei Einsätze (7) im hinteren Teil der Sohle (3) des Basisteils (2) angeordnet sind, und zwei weitere Einsätze (7) im vorderen Teil dieser Sohle (3) angeordnet sind, wobei diese Einsätze (7) jeweils vier Befestigungsschrauben (27) der Metallplatte (8) zu Versteifung aufnehmen.

5. Postoperatives Schuhwerk nach einem der Ansprüche 1 bis 4, **dadurch gekennzeichnet, dass** die Metallplatte (8) zur Versteifung eine einzige Führungsbahn in Form eines in Längsrichtung verlaufenden Schlitzes (12) aufweist, der sich im wesentlichen entlang der Mittelachse dieser Metallplatte (8) erstreckt, wobei der Keil (13) zur Entlastung des Vorderfußes und das Pufferteil (16) mit abgerundetem Profil jeweils mittels zweier Schrauben (28, 29) befestigt werden, die in diesem längslaufenden Schlitz (12) sitzen.

6. Postoperatives Schuhwerk nach einem der Ansprüche 1 bis 5, **dadurch gekennzeichnet, dass** die Metallplatte (8) zur Versteifung auf ihrer Unterseite einen Überzug aus gummiartigem Material hat.

7. Postoperatives Schuhwerk nach Anspruch 4, **dadurch gekennzeichnet, dass** die Befestigung des Absatzes (19) mittels Schrauben (27) durchgeführt wird, die in die Einsätze(7) mit Innengewinde geschraubt werden, die im hinteren Teil der Sohle (3) des Basisteils (2) vorgesehen sind.

8. Postoperatives Schuhwerk nach einem der Ansprüche 1 bis 7, **dadurch gekennzeichnet, dass** die Zubehörteile, nämlich der Keil (13) zur Entlastung des Vorderfußes, das abgerundete Pufferteil (16) und der Absatz (19), aus einem relativ starren Kunststoff, insbesondere Polypropylen, hergestellt sind.

9. Postoperatives Schuhwerk nach einem der Ansprüche 1 bis 8, **dadurch gekennzeichnet, dass** es als ergänzendes lösbares Zubehörteil eine Anstoßblende (22) umfasst, die vorne am Basisteil (2) befestigt ist, um den Vorderfuß gegen Stöße zu schützen.

10. Postoperatives Schuhwerk nach der Gesamtheit der Ansprüche 4 und 9, **dadurch gekennzeichnet, dass** die Einstellung der lösbaren Anstoßblende (22) mittels Schrauben (27) durchgeführt wird, die in die Einsätze (7) mit Innengewinde geschraubt werden, die im Vorderteil der Sohle (3) des Basisteils (2) vorgesehen sind.

11. Postoperatives Schuhwerk nach einem der Ansprüche 1 bis 10, **dadurch gekennzeichnet, dass** es noch eine herausnehmbare verbreiterte Innensohle (26) umfasst, die über der Sohle (3) des Basisteils (2) sitzt.
